# EUROPEAN PATENT APPLICATION

(11) **EP 4 767 945 A1**
(43) Date of publication of application: **01.07.2026**
(21) Application number: 25224656.6
(22) Date of filing: 17.12.2025
(51) Int. Cl.: A61B 6/00, A61B 6/46, A61B 6/58, A61B 6/42

(54) **IMAGING SUPPORT APPARATUS, IMAGING SUPPORT METHOD, IMAGING SUPPORT PROGRAM, AND RADIOGRAPHIC IMAGING APPARATUS**

(30) Priority: 27.12.2024 JP 2024232512
(71) Applicant: FUJIFILM Corporation, Tokyo 106-8620 (JP)
(72) Inventor: HORIUCHI, Hisatsugu, Tokyo, 106-8620 (JP)
(74) Representative: Dehns Germany Partnerschaft mbB

(57) **Abstract**

In order to support imaging of a radiographic imaging apparatus including a radiation source that emits radiation, and an optical camera (21) that is mounted on the radiation source and that captures an optical image (G1) of a subject in a direction from the radiation source, a processor (41) derives a relative angle between the radiation source and a target plane; and displays the optical image (G1) and an angle indicator representing the relative angle and a target angle on a display (25) mounted on the radiation source.

## Description

### BACKGROUND

### Technical Field

The present disclosure relates to an imaging support apparatus, an imaging support method, an imaging support program, and a radiographic imaging apparatus.

### Related Art

A radiation image of a patient is captured using a radiation detector at a ward-round destination using a mobile radiographic imaging apparatus (ward-round cart). In a case where the radiographic imaging is performed at the ward-round destination in this way, it is required to align a radiation source and the radiation detector. Specifically, it is required to perform the alignment of the radiation source by adjusting a relative position and a relative angle between the radiation source and the radiation detector such that a center of radiation emitted from the radiation source matches an imaging center of a subject and an optical axis of the radiation intersects the radiation detector perpendicularly. Therefore, a method of providing an optical camera in the vicinity of a radiation source, displaying an image acquired by the optical camera on goggles worn by an operator, and displaying a graphic for guiding a position of the radiation source, such as a relative angle between the radiation source and a radiation detector and an imaging center position, on a display screen has been proposed (see JP2023-019747A).

In addition, a method of providing an optical camera and a display in a portable X-ray source that is operated by being held in a hand of an operator, displaying an optical image acquired by the optical camera on the display, and numerically displaying a relative angle between a radiation source and a radiation detector has been proposed (see JP2016-209548A).

However, in the ward-round cart, the radiation source mounted on an arm may be rotated around the optical axis of the radiation to perform the alignment. In a case where the radiation source is rotated around the optical axis, the optical camera mounted on the radiation source is also rotated, and thus the acquired optical image is also rotated. Therefore, in a case where the optical image is displayed on the goggles as described in JP2023-019747A, an up-down direction of the displayed optical image may not match an up-down direction of a subject that is being viewed by the operator. In this way, in a case where the up-down direction of the optical image does not match the up-down direction of an actual subject, a sense of incongruity occurs in a case of positioning the radiation source.

### SUMMARY OF THE INVENTION

The present disclosure has been made in view of the above-described circumstances, and an object thereof is to enable the alignment between the radiation source and a target plane such as the radiation detector without a sense of incongruity.

The present disclosure relates to an imaging support apparatus for a radiographic imaging apparatus including a radiation source that emits radiation, and an optical camera that is mounted on the radiation source and that captures an optical image in a direction from the radiation source toward a subject, the imaging support apparatus comprising: a display mounted on the radiation source; and a processor, in which the processor is configured to: derive a relative angle between the radiation source and a target plane; and display the optical image and an angle indicator representing the relative angle and a target angle on the display.

In the imaging support apparatus according to the present disclosure, the display may be mounted on the radiation source so as to rotate together with the optical camera.

The imaging support apparatus according to the present disclosure may further comprise: a first angle sensor that measures an angle of the radiation source; and a second angle sensor that measures an angle of the target plane, in which the processor is configured to derive the relative angle based on a difference between the angle of the radiation source measured by the first angle sensor and the angle of the target plane measured by the second angle sensor.

In the imaging support apparatus according to the present disclosure, the processor may be configured to: from a sensor that is provided on the radiation source and that acquires distance information representing an imaging distance in a direction from the radiation source toward the subject, acquire the distance information; and derive the relative angle based on a difference between the imaging distances at a plurality of positions in the direction from the radiation source toward the subject.

In the imaging support apparatus according to the present disclosure, the target plane may be a detection surface of a radiation detector that detects radiation transmitted through the subject to acquire a radiation image of the subject.

In the imaging support apparatus according to the present disclosure, the target plane may be a surface of a patient table on which the subject is placed.

In the imaging support apparatus according to the present disclosure, the angle indicator may include a graphic for matching the relative angle to the target angle.

In the imaging support apparatus according to the present disclosure, the angle indicator may further include an adjustment value for matching the relative angle to the target angle.

In the imaging support apparatus according to the present disclosure, the graphic may include a first indicator representing the target angle and a second indicator representing the relative angle, and the processor may be configured to change a position of the second indicator relative to a position of the first indicator in accordance with the relative angle.

In the imaging support apparatus according to the present disclosure, the graphic may include a first linear graphic and a second linear graphic respectively for a vertical direction and a horizontal direction of the optical image, and the processor may be configured to display the first linear graphic and the second linear graphic on edges of the optical image in the vertical direction and the horizontal direction.

In the imaging support apparatus according to the present disclosure, the graphic may have a predetermined reference shape, and the processor may be configured to change the reference shape in accordance with the relative angle.

In the imaging support apparatus according to the present disclosure, the processor may be configured to further display an imaging center indicator representing an imaging center of the subject and an irradiation center indicator representing a center position of the radiation emitted from the radiation source on the display.

The present disclosure relates to an imaging support method for a radiographic imaging apparatus including a radiation source that emits radiation, and an optical camera that is mounted on the radiation source and that captures an optical image in a direction from the radiation source toward a subject, the imaging support method being executed by a computer, the imaging support method comprising: deriving a relative angle between the radiation source and a target plane; and displaying the optical image and an angle indicator representing the relative angle and a target angle on a display mounted on the radiation source.

The present disclosure relates to an imaging support program for a radiographic imaging apparatus including a radiation source that emits radiation, and an optical camera that is mounted on the radiation source and that captures an optical image in a direction from the radiation source toward a subject, the imaging support program causing a computer to execute: a procedure of deriving a relative angle between the radiation source and a target plane; and a procedure of displaying the optical image and an angle indicator representing the relative angle and a target angle on a display mounted on the radiation source.

It should be noted that the disclosed technology may be applied to a program product.

The present disclosure relates to a radiographic imaging apparatus comprising: a radiation source that emits radiation; a sensor that acquires information representing an imaging distance in a direction from the radiation source toward a subject; an optical camera that captures an optical image in the direction from the radiation source toward the subject; and the imaging support apparatus according to the present disclosure.

The radiographic imaging apparatus according to the present disclosure may further comprise: a body that is movable; and an arm that is foldable and that connects the body to the radiation source.

According to the present disclosure, angular alignment between the radiation source and the target plane can be performed without a sense of incongruity.

### BRIEF DESCRIPTION OF THE DRAWINGS

FIG. 1 is an external perspective view of a radiographic imaging apparatus to which an imaging support apparatus according to the present embodiment is applied.
FIG. 2 is a diagram illustrating a state where the radiographic imaging apparatus according to the present embodiment is used.
FIG. 3 is a diagram illustrating a detailed configuration of a radiation source unit.
FIG. 4 is a diagram illustrating a hardware configuration of the imaging support apparatus according to the present embodiment.
FIG. 5 is a functional block diagram of the imaging support apparatus according to the present embodiment.
FIG. 6 is a diagram illustrating a display screen displayed on a display.
FIG. 7 is a flowchart illustrating processing performed in the present embodiment.
   FIG. 8 is a diagram illustrating a display screen displayed on the display.
FIG. 9 is a diagram illustrating a display screen displayed on the display.
FIG. 10 is a diagram illustrating a display screen displayed on the display.
FIG. 11 is a diagram illustrating a display screen displayed on the display.
FIG. 12 is a diagram illustrating a display screen displayed on the display.
FIG. 13 is a diagram illustrating a display screen displayed on the display.
FIG. 14 is a diagram illustrating a display screen displayed on the display.
FIG. 15 is a diagram illustrating a display screen displayed on the display.
FIG. 16 is a diagram illustrating a display screen displayed on the display.
FIG. 17 is a diagram illustrating a display screen displayed on the display.
FIG. 18 is a diagram illustrating a display screen displayed on the display.
FIG. 19 is a diagram illustrating a display screen displayed on the display.
FIG. 20 is a diagram illustrating a display screen displayed on the display.
FIG. 21 is a diagram illustrating a display screen displayed on the display.
FIG. 22 is a diagram illustrating plane detection.

### DETAILED DESCRIPTION

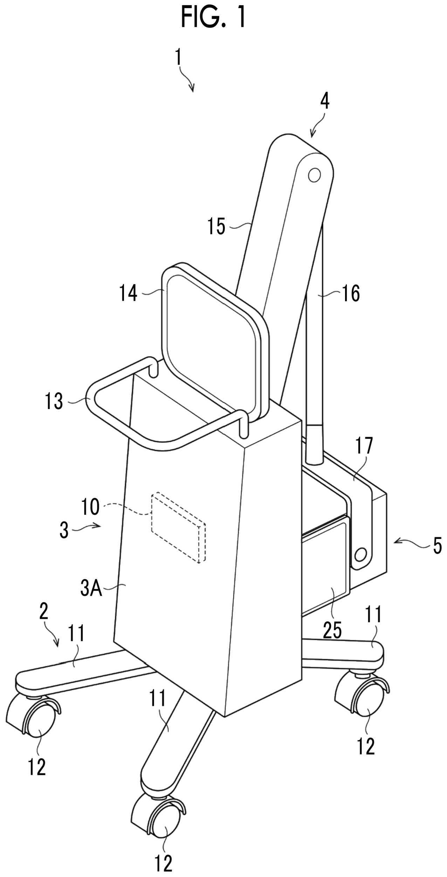

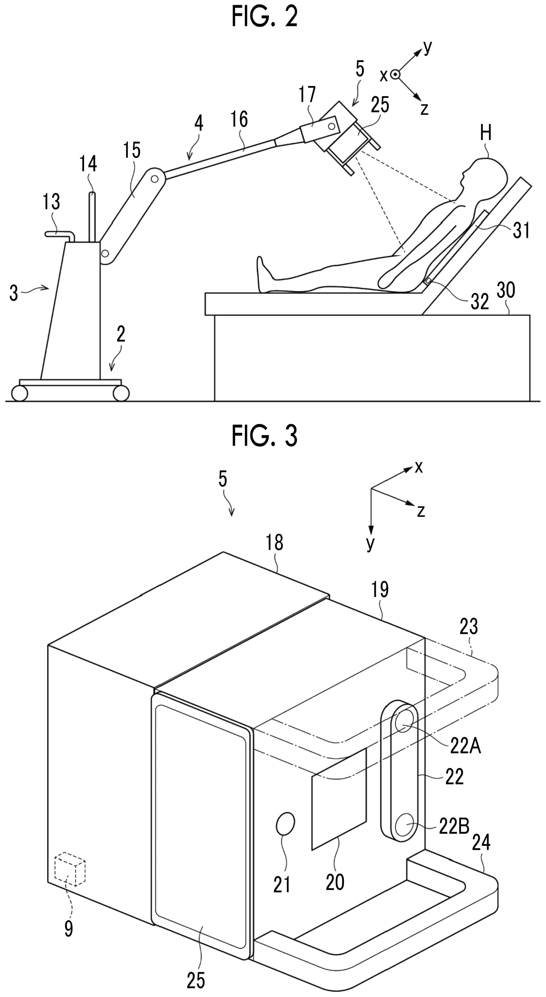

Hereinafter, an embodiment of the present disclosure will be described with reference to the accompanying drawings. FIG. 1 is an external perspective view of a radiographic imaging apparatus to which an imaging support apparatus according to the present embodiment is applied, and FIG. 2 is a diagram illustrating a state where the radiographic imaging apparatus according to the present embodiment is used. A radiographic imaging apparatus 1 to which the imaging support apparatus according to the present embodiment is applied is a ward-round cart type radiographic imaging apparatus, and includes a leg part 2 that is movable on an apparatus placement surface, a body 3 that is supported on the leg part 2, an arm 4 that is connected to the body 3, and a radiation source unit 5 that is mounted on a distal end portion of the arm 4.

The leg part 2 includes four legs 11 and wheel parts 12 mounted on lower surfaces of distal end portions of the legs 11. A stopper (not illustrated) is provided in the wheel part 12 such that the wheels do not rotate unintentionally.

The body 3 accommodates a computer 10, a battery, and the like for controlling the radiographic imaging apparatus 1 in a housing 3A. The computer 10 includes the imaging support apparatus according to the present embodiment. A handle 13 for pushing or pulling the radiographic imaging apparatus is mounted on an upper end of the housing 3A. An operation panel 14 is mounted on an upper portion of the housing 3A.

As the operation panel 14, a touch panel type is adopted in which a display is integrated, and the operation panel 14 receives an instruction of an operator, such as setting of imaging conditions and imaging start, and inputs the instruction to the radiographic imaging apparatus 1. As an imaging menu, chest imaging, extremity imaging, upper-body imaging, and the like can be set.

The arm 4 consists of a first member 15 and a second member 16 that are foldable. The first member 15 is connected to the body 3 so as to be rotatable in an up-down direction. The first member 15 and the second member 16 are connected so as to be rotatable relative to each other.

The radiation source unit 5 is mounted on a distal end of the second member 16 of the arm 4 by a mounting member 17. The mounting member 17 supports the radiation source unit 5 to be swingable. The mounting member 17 is mounted so as to be rotatable around a major axis of the second member 16.

In a case where the radiographic imaging apparatus 1 is used, for example, as illustrated in FIG. 2, an upper body of a subject H is raised on a patient table 30, and a radiation detector 31 for generating a radiation image in which radiation transmitted through the subject H is detected is inserted between a raised portion of the patient table 30 and the subject H. The operator moves the radiographic imaging apparatus 1 close to the patient table 30, unfolds the arm 4 that is in a folded state, and moves the radiation source unit 5 to perform alignment such that a predetermined part of the subject H is irradiated with the radiation at the set SID and the radiation is emitted perpendicularly to the radiation detector 31. The alignment of the radiation source unit 5 will be described later.

The radiation detector 31 is a cassette type detector configured to acquire the radiation image of the subject H by detecting the radiation. Further, the radiation detector 31 is a wireless detector, and transmits the radiation image acquired by the irradiation with the radiation to the computer 10 wirelessly.

In the present embodiment, the radiation detector 31 includes a motion sensor 32. The motion sensor 32 is a nine-axis motion sensor that detects three-axis acceleration, three-axis angular velocity, and three-axis tilt of the radiation detector 31. The acceleration, the angular velocity, and the tilt detected by the motion sensor 32 are output to the computer 10 as movement information. The processing using the movement information will be described later. The motion sensor 32 is an example of a second angle sensor according to the present disclosure.

FIG. 3 is a diagram illustrating a detailed configuration of the radiation source unit. As illustrated in FIG. 3, the radiation source unit 5 includes a tube housing part 18 that accommodates a radiation tube such as an X-ray tube, and a collimator 19 that is mounted on the tube housing part 18 so as to be rotatable around an optical axis of the radiation. An emission window 20 for radiation, an optical camera 21, a stereo camera 22, and two handles 23 and 24 are mounted on a radiation emission surface of the collimator 19. A display 25 is mounted on a side surface of the collimator 19. In FIG. 3, the handle 23 is illustrated in phantom for illustrating a configuration of the collimator 19. A motion sensor 9 is mounted on the radiation source unit 5.

The collimator 19 sets an irradiation field of the radiation by changing a size of the emission window 20. The irradiation field is set in response to the instruction from the operation panel 14. An irradiation field lamp that is a visible light source is mounted inside the emission window 20 of the collimator 19. By turning on the irradiation field lamp, the subject is irradiated with visible light, and an irradiation range of the visible light changes in accordance with the size of the emission window 20. As a result, the operator can check the radiation irradiation field on the subject H.

The optical camera 21 acquires an optical image G1 in a direction in which the radiation is emitted from the radiation source unit 5. The optical image G1 is a moving image at a predetermined frame rate in which an object on a side irradiated with the radiation from the radiation source unit 5 is represented by RGB pixels. The acquired optical image G1 is displayed on the display 25 as will be described later. The optical camera 21 is configured to acquire a color optical image G1, but may acquire a monochrome optical image G1.

The stereo camera 22 includes two cameras 22A and 22B, and acquires an imaging distance image G2 by measuring a distance based on the principle of triangulation. The imaging distance image G2 is also a moving image at a predetermined frame rate. In the imaging distance image G2, each pixel represents an imaging distance in a direction from the radiation source unit 5 toward the subject. A time-of-flight (TOF) camera that measures a distance by a time for light to return may be used instead of the stereo camera 22. The imaging distance image may be derived by using a light detection and ranging (LiDAR) sensor. The stereo camera 22, the TOF camera, and the LiDAR sensor are examples of a sensor that acquires distance information representing an imaging distance according to the present disclosure. The imaging distance image G2 is an example of distance information representing an imaging distance according to the present disclosure. The distance information is not limited to an image format, and may be a numerical value representing the imaging distance itself.

The handles 23 and 24 are used by the operator to grip and adjust a position and an angle of the radiation source unit 5. Here, in a case where an x-axis, a y-axis, and a z-axis are set as illustrated in FIG. 3, the collimator 19 is mounted on the tube housing part 18 so as to be rotatable around the z-axis. Therefore, the irradiation field of the radiation for the subject H can be rotated. In addition, as illustrated in FIG. 1, the radiation source unit 5 is mounted on the second member 16 so as to be rotatable around the major axis of the second member 16 and is mounted on the second member 16 so as to be swingable by the mounting member 17. Therefore, the angles around the x-axis and the y-axis illustrated in FIG. 3 can be adjusted.

The optical image G1 captured by the optical camera 21 is displayed on the display 25. The display content on the display 25 will be described later. The display 25 is mounted on the collimator 19. Therefore, the display 25 is mounted on the radiation source, that is, the tube housing part 18 so as to rotate together with the optical camera 21.

The motion sensor 9 is a nine-axis motion sensor that detects three-axis acceleration, three-axis angular velocity, and three-axis tilt of the radiation source unit 5. The acceleration, the angular velocity, and the tilt detected by the motion sensor 9 are output to the computer 10 as movement information. The motion sensor 9 is an example of a first angle sensor according to the present disclosure. The processing using the movement information will be described later.

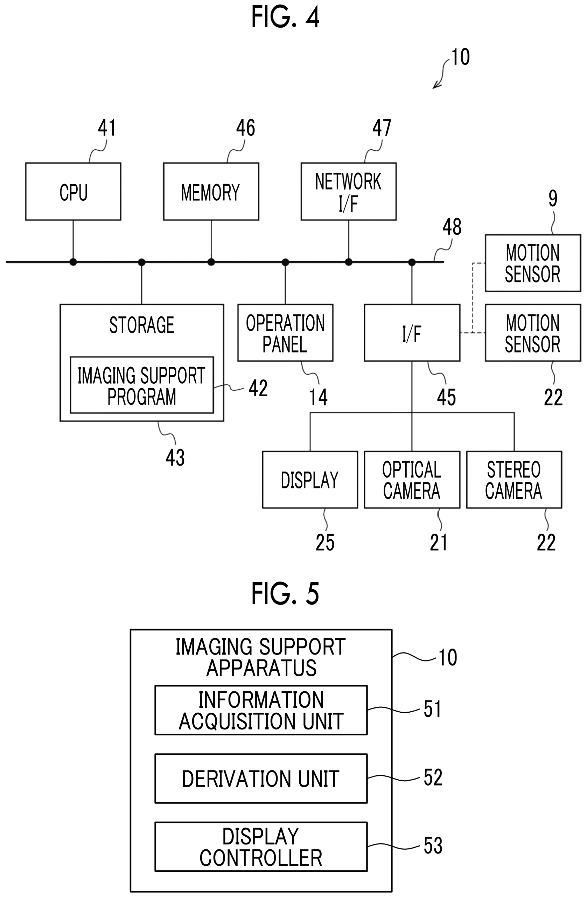

Hereinafter, the computer for executing processing of the imaging support apparatus according to the present embodiment will be described. FIG. 4 is a diagram illustrating a hardware configuration of the computer for executing the processing of the imaging support apparatus. As illustrated in FIG. 4, the computer 10 includes a central processing unit (CPU) 41, a non-volatile storage 43, and a memory 46 as a temporary storage area. In addition, the computer 10 includes the operation panel 14, a network interface (I/F) 47 that is connected to a network (not illustrated), and a wired and wireless I/F 45 for connecting the optical camera 21, the stereo camera 22, and the display 25 to the computer 10. The motion sensor 9 of the radiation source unit 5 and the motion sensor 32 of the radiation detector 31 are connected to the computer 10 by wireless communication via the I/F 45. The CPU 41, the storage 43, the operation panel 14, the I/F 45, the memory 46, and the network I/F 47 are connected to a bus 48. The CPU 41 is an example of a processor according to the present disclosure.

The computer 10 performs processing of displaying the radiation image acquired by the radiation detector 31 and transmitting the radiation image to an external apparatus or the like, but detailed description of these types of processing will be omitted here. Further, the computer 10 includes the imaging support apparatus according to the present embodiment. Therefore, in the following description, the imaging support apparatus according to the present embodiment will also be denoted by reference numeral 10.

The storage 43 is implemented by a hard disk drive (HDD), a solid state drive (SSD), a flash memory, and the like. An imaging support program 42 is stored in the storage 43 as a storage medium. The CPU 41 reads out the imaging support program 42 from the storage 43, loads the readout imaging support program 42 into the memory 46, and executes the loaded imaging support program 42.

Hereinafter, a functional configuration of the imaging support apparatus according to the present embodiment will be described. FIG. 5 is a diagram illustrating the functional configuration of the imaging support apparatus according to the present embodiment. As illustrated in FIG. 5, the imaging support apparatus 10 comprises an information acquisition unit 51, a derivation unit 52, and a display controller 53. In a case where the CPU 41 executes the imaging support program 42, the CPU 41 functions as the information acquisition unit 51, the derivation unit 52, and the display controller 53.

The information acquisition unit 51 acquires the optical image G1 acquired by the optical camera 21. Further, the information acquisition unit 51 also acquires the movement information output by the motion sensors 9 and 32.

The derivation unit 52 derives a relative angle between the radiation source unit 5 and a target plane. In the present embodiment, the target plane is a detection surface of the radiation detector 31. The derivation unit 52 derives the relative angle between the radiation source unit 5 and the target plane based on the movement information acquired from each of the motion sensor 9 of the radiation source unit 5 and the motion sensor 32 of the radiation detector 31.

Here, in the present embodiment, the radiographic imaging apparatus 1 is moved to a leg side of the subject H with respect to the patient table 30 as illustrated in FIG. 2. In such a situation, the derivation unit 52 acquires a rotation angle of the radiation source unit 5 around the x-axis and the y-axis and a rotation angle of the radiation detector 31 around the x-axis and the y-axis from the motion sensor 9 of the radiation source unit 5 and the motion sensor 32 of the radiation detector 31, respectively. Then, relative angles αx and αy of the radiation source unit 5 and the radiation detector 31 around the x-axis and the y-axis are derived. For example, in a case where the rotation angle of the radiation source unit 5 around the x-axis is 8.2° and the rotation angle of the radiation detector 31 around the x-axis is 10°, the relative angle αx = 10 - 8.2 = 1.8° is derived by calculation.

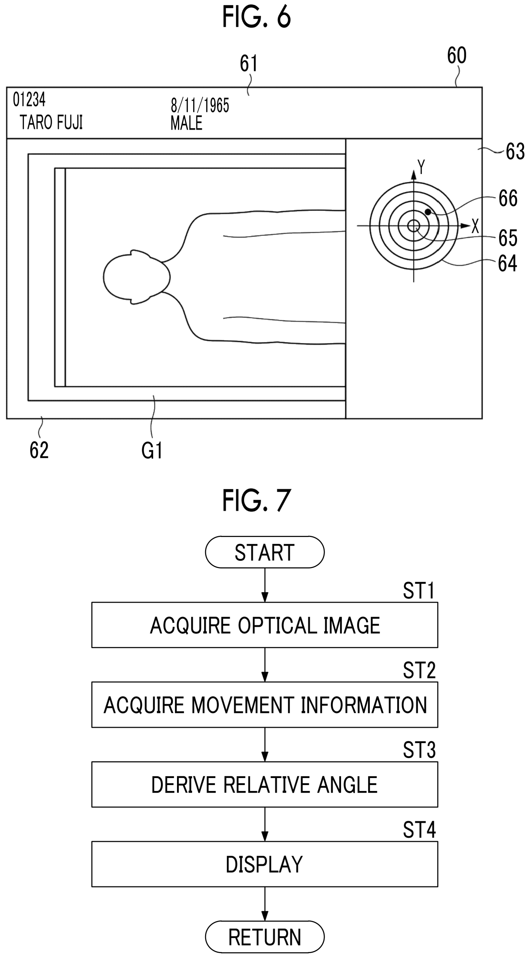

The display controller 53 displays a display screen including an angle indicator representing the relative angle derived by the derivation unit 52 and a target angle and the optical image G1 on the display 25. FIG. 6 is a diagram illustrating the display screen on the display 25. As illustrated in FIG. 6, a display screen 60 includes an information region 61 that displays information on the subject H, an image region 62 that displays the optical image G1, and an indicator region 63 that displays the angle indicator.

A name, a patient number, a birthday, a gender, and the like of the subject H are displayed in the information region 61. The optical image G1 is displayed in the image region 62.

A graphic 64 for matching the relative angle to the target angle is displayed in the indicator region 63. The graphic 64 consists of a plurality of concentric circles with different diameters. In the graphic 64, a center region 65 that is the smallest circle represents the target angle, and the relative angle increases as a distance from the center region 65 increases. Further, the graphic 64 includes a mark 66 representing the relative angle. In FIG. 6, the mark is represented by a black circle, but the present disclosure is not limited to this. The center region 65 is an example of a first indicator according to the present disclosure, and the mark 66 is an example of a second indicator according to the present disclosure.

The operator can perform angular alignment of the radiation source unit 5 to the radiation detector 31 by changing the angle of the radiation source unit 5 such that the mark 66 is moved to the center region 65 using the handles 23 and 24 of the radiation source unit 5 while viewing the display screen 60.

After the angular alignment, the operator operates the operation panel 14 to turn on the irradiation field lamp and irradiates the subject H with the visible light representing the irradiation field. Then, after the irradiation field is adjusted, an instruction to perform the imaging is issued from the operation panel 14, the imaging of the subject H is performed, and thus the radiation image is acquired.

Hereinafter, processing performed in the present embodiment will be described. FIG. 7 is a flowchart illustrating the processing performed in the present embodiment. First, the information acquisition unit 51 acquires the optical image G1 acquired by the optical camera 21 (step ST1). The derivation unit 52 acquires the movement information from each of the motion sensor 9 of the radiation source unit 5 and the motion sensor 32 of the radiation detector 31 (step ST2), and derives the relative angle between the radiation source unit 5 and the detection surface of the radiation detector 31 (step ST3). The display controller 53 displays the optical image G1 and the angle indicator representing the relative angle and the target angle on the display 25 (step ST4), and returns to step ST1.

In the present embodiment, as described above, the relative angle between the radiation source unit 5 and the detection surface of the radiation detector 31 is displayed on the display 25 together with the optical image G1. Here, the display 25 is mounted on the collimator 19 of the radiation source unit 5 so as to rotate together with the optical camera 21. Therefore, in a case where the alignment is performed such that the radiation source unit 5 is rotated around the optical axis of the radiation, a relationship between an up-down direction of the optical image G1 displayed on the display 25 and an up-down direction of the optical camera 21 is easily understood. As a result, in the present embodiment, the alignment between the radiation source unit 5 and the radiation detector 31 can be performed without a sense of incongruity.

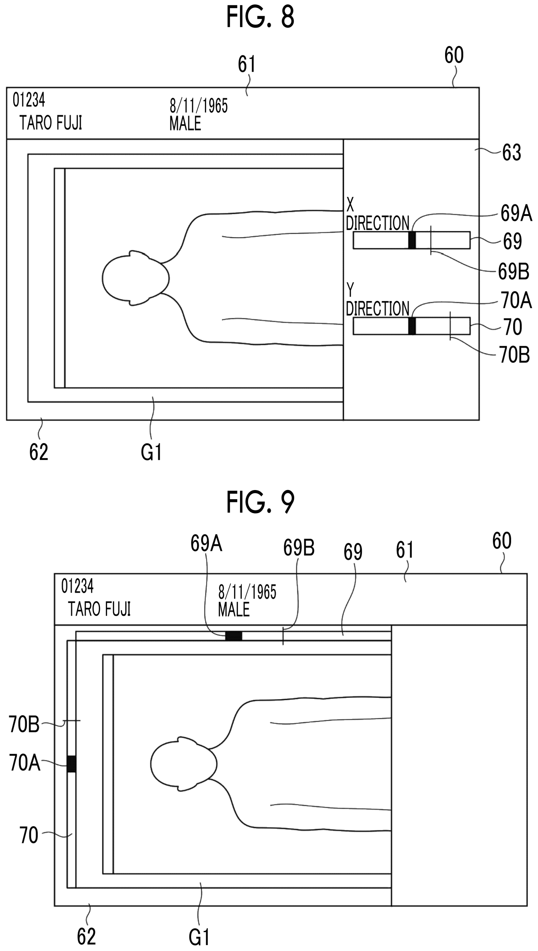

In the above-described embodiment, the concentric graphic 64 illustrated in FIG. 6 is displayed as the angle indicator, but the present disclosure is not limited to this. As illustrated in FIG. 8, linear graphics 69 and 70 representing the target angle and the relative angle in each of the X direction and the Y direction may be displayed as the angle indicator. In the graphic 69, a mark 69A representing the target angle and a line 69B representing the relative angle in the X direction are displayed. In the graphic 70, a mark 70A representing the target angle and a line 70B representing the relative angle in the Y direction are displayed. In FIG. 8, the marks 69A and 70A are examples of a first indicator according to the present disclosure, and the lines 69B and 70B are examples of a second indicator according to the present disclosure. The X direction is a horizontal direction of the displayed optical image G1, and the Y direction is a vertical direction of the optical image G1.

In this case, the operator can perform the angular alignment of the radiation source unit 5 to the radiation detector 31 by changing the angle of the radiation source unit 5 such that the lines 69B and 70B match the marks 69A and 70A within a range in each of the graphics 69 and 70 using the handles 23 and 24 of the radiation source unit 5 while viewing the display screen 60. The range of the marks 69A and 70A may be, for example, about ±2°.

As illustrated in FIG. 9, the linear graphics 69 and 70 may be displayed on an upper side (that is, a horizontal edge) and a left side (that is, a vertical edge) of the optical image G1.

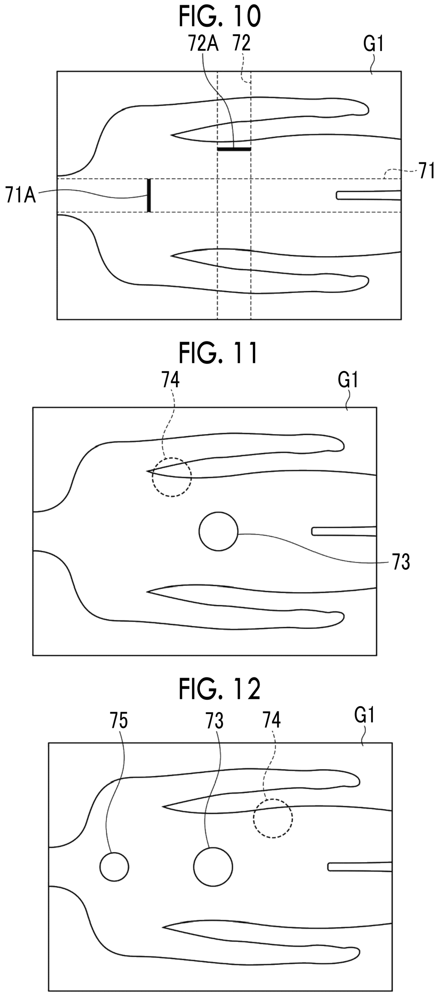

In addition, as illustrated in FIG. 10, a region 71 having a predetermined width that vertically bisects the optical image G1 and a region 72 having a predetermined width that horizontally bisects the optical image G1 may be set, a bar 71A representing the relative angle in the X direction may be displayed in the region 71, and a bar 72A representing the relative angle in the Y direction may be displayed in the region 72. In this case, the bars 71A and 72A move linearly in the regions 71 and 72 in accordance with the relative angle between the radiation source unit 5 and the radiation detector 31. In a case where the relative angle matches the target angle, the bars 71A and 72A intersect each other, and a cross-shaped graphic is displayed in a region in which the regions 71 and 72 intersect each other. In FIG. 10, the bars 71A and 72A are examples of an angle indicator according to the present disclosure, specifically, examples of a graphic for matching the relative angle to the target angle.

A target position during the imaging of the subject H may be detected from the optical image G1, and an indicator representing the target position may be displayed on the optical image G1. For example, as illustrated in FIG. 11, a ring-shaped indicator 73 that surrounds the target position may be displayed. In this case, a ring-shaped indicator 74 having the same shape as the indicator 73 may be displayed as the indicator representing the relative angle. The indicator 74 may be different from the indicator 73 in color, density, or the like such that it is clear that the indicator 74 represents the relative angle. In FIG. 11, the indicators 73 and 74 are the ring-shaped graphics, but the indicators 73 and 74 may be any shape graphics such as a polygon and a star shape in addition to the circular shape. In this case, by making the color of the indicator 73 different from the color of the indicator 74, the color of the region in which the indicators 73 and 74 overlap each other can be set to a mixed color of the indicators 73 and 74, and thus it is possible to easily check that the relative angle matches the target angle. In this case, the indicator 73 is an example of a first indicator according to the present disclosure, and the indicator 74 is an example of a second indicator according to the present disclosure.

Further, the indicator representing the target position and the angle indicator may be displayed separately. For example, as illustrated in FIG. 12, a ring-shaped indicator 75 representing the target position may be displayed in addition to the indicators 73 and 74. In this case, first, the angular alignment between the radiation source unit 5 and the radiation detector 31 may be performed such that the indicators 73 and 74 match each other, and then the radiation source unit 5 may be moved in parallel to the radiation detector 31 such that the overlapping indicators 73 and 74 match the indicator 75 representing the target position.

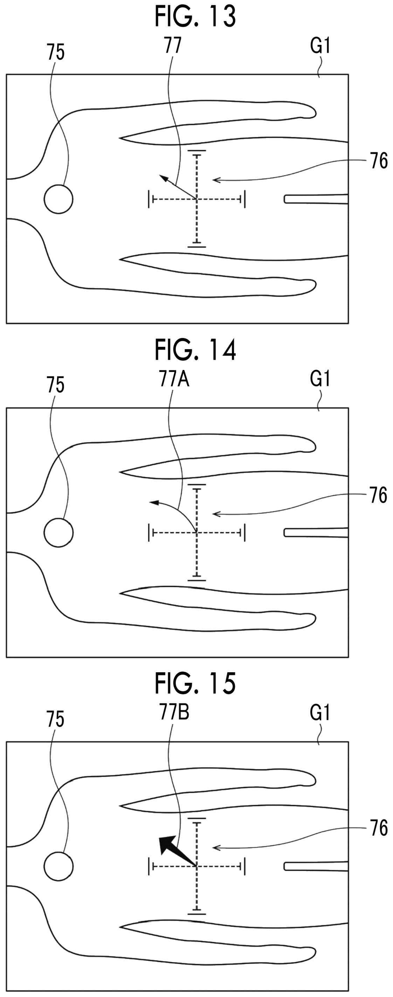

In addition, as illustrated in FIG. 13, a frame 76 that defines the center of the emitted radiation may be displayed, and the angle indicator may be displayed at the center of the frame 76. In FIG. 13, as the angle indicator, an arrow 77 is displayed. A direction of the arrow 77 represents a direction in which the relative angle is tilted, and a length or a thickness of the arrow 77 represents a magnitude of the relative angle. The arrow 77 is an example of an angle indicator according to the present disclosure. The frame 76 is an example of an irradiation center indicator representing a center position of the radiation emitted from the radiation source according to the present disclosure. In this case, the indicator 75 is an example of an imaging center indicator representing an imaging center of the subject.

In a case where the arrow 77 illustrated in FIG. 13 is displayed, an arrow 77A curved as illustrated in FIG. 14 may be displayed such that a direction of angle change is clear instead of the parallel movement. In addition, as illustrated in FIG. 15, an arrow 77B of which a width increases as a distance from the indicator 75 increases may be displayed.

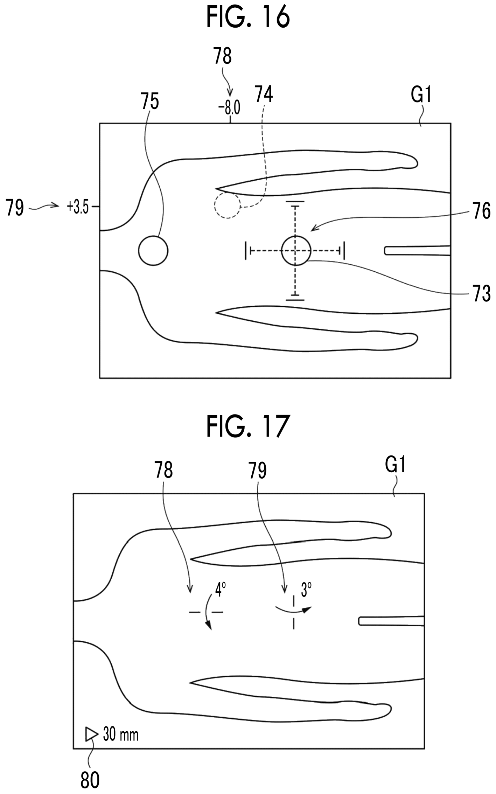

As illustrated in FIG. 16, the indicators 73, 74, and 75 and the frame 76 may be displayed. Furthermore, a numerical value 78 representing the relative angle in the X direction may be displayed on the upper side of the optical image G1, and a numerical value 79 representing the relative angle in the Y direction may be displayed on the left side. In this case, the arrows 77, 77A, and 77B illustrated in FIGS. 13 to 15 may be displayed instead of the indicators 73 and 74.

In addition, as illustrated in FIG. 17, icons 78 and 79 representing a rotation direction of the angle may be displayed, and the magnitude of the relative angle may be displayed on the icons 78 and 79. The icons 78 and 79 are examples of an angle indicator according to the present disclosure. In this case, an icon 80 representing a parallel movement amount for matching the target position of the subject H to the imaging center may be displayed together with a numerical value representing a movement amount.

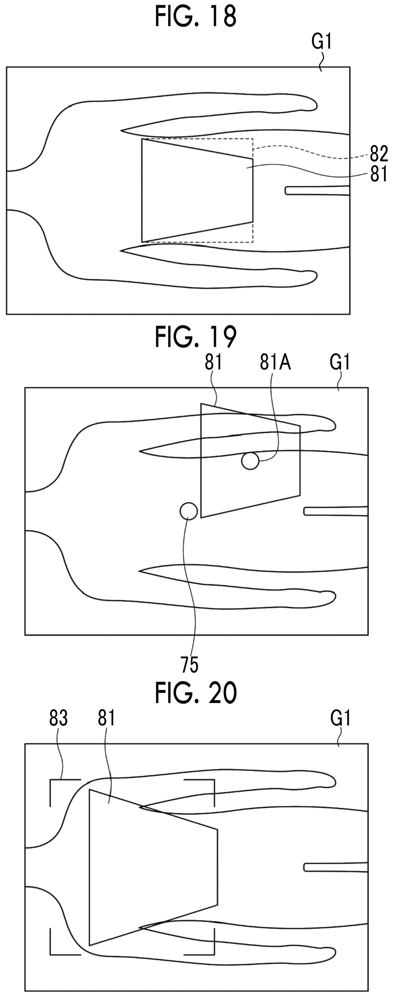

In addition, as illustrated in FIG. 18, a semi-transparent rectangular graphic that is deformed in accordance with the relative angle may be displayed as the angle indicator. For example, in a case where the relative angle matches the target angle, a rectangular graphic 82 may be displayed, and the rectangle may be deformed such that a side in the tilted direction is shortened in a case where the relative angle increases. The rectangular graphic 82 is an example of a reference shape according to the present disclosure. The graphic 82 is semi-transparent, but may be a transparent graphic having only a contour or an opaque graphic. In FIG. 18, the fact that the relative angles in the up-down direction match each other and the tilt remains in the relative angle in the left-right direction is indicated by displaying a trapezoidal graphic 81 having a short right side. In a case where the tilt of the relative angle in the left-right direction is reversed from FIG. 18, a trapezoid having a short left side is displayed.

In addition, in a case where the relative angles in the left-right direction match each other and the tilt remains in the relative angle in the up-down direction, a trapezoid having a short upper side or a short lower side is displayed in accordance with the direction of the tilt. In a case where the tilt remains in the relative angle in both the up-down direction and the left-right direction, a distorted quadrangle is displayed instead of the trapezoid.

It is preferable that the deformation of the rectangular graphic is performed in a discrete manner. For example, it is preferable that the degree of deformation is increased stepwise in five stages of 0° to 5°, 5° to 7°, 7° to 9°, 9° to 11°, and 11° or more in the relative angle. As a result, flickering in a case where the rectangle is deformed can be prevented, and thus the angle indicator can be easily seen. The degree of deformation of the graphic may be performed in one stage. For example, the rectangular graphic may not be deformed in a case where the relative angle is less than a threshold value (for example, less than 5°), and the graphic may be deformed in a case where the relative angle is equal to or greater than the threshold value (for example, 5° or more). In this case, it is possible to check whether the relative angle matches the target angle in accordance with whether the rectangular graphic is deformed.

In addition, as illustrated in FIG. 19, the indicator 75 representing the target position of the subject H during the imaging may be displayed, and an indicator 81A representing a centroid of the graphic 81 may be displayed. In this case, the alignment in the parallel direction can be performed by performing the alignment of the relative angle such that the graphic 81 becomes a rectangle, and moving the radiation source unit 5 such that the indicator 81A representing the centroid of the graphic 81 matches the indicator 75.

In addition, as illustrated in FIG. 20, marks 83 representing four corners of the irradiation field may be displayed as the target position of the subject H during the imaging. In this case, it is preferable that a size of a region surrounded by the marks 83 matches a size of the graphic 81, and a color is further applied to the graphic 81. As a result, the alignment between the radiation source unit 5 and the radiation detector 31 can be performed by moving the radiation source unit 5 such that the graphic 81 matches the region surrounded by the marks 83.

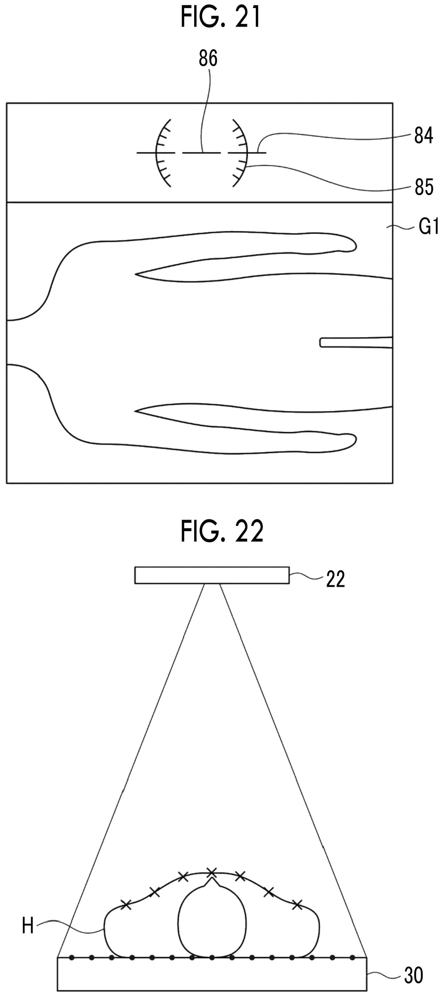

In addition, as illustrated in FIG. 21, an indicator 84 representing the relative angle in the X direction and an indicator 85 representing the relative angle in the Y direction may be displayed. In this case, the indicators 84 and 85 represent the relative angles of the radiation source unit 5 in the X direction and the Y direction instead of the relative angle of the image captured by the optical camera 21. In this case, in the indicator 84, in a case where the relative angle in the X direction changes, a bar 86 moves up and down. In the indicator 85, in a case where the relative angle in the Y direction changes, the bar 86 rotates around a center point of the indicator 85. As a result, it is possible to represent the relative angles of the radiation source unit 5 in the X direction and the Y direction.

In the above-described embodiment, the target plane is the detection surface of the radiation detector 31, but the present disclosure is not limited to this. A surface of the patient table 30 may be used as the target plane. In such a case, the relative angle between the radiation source unit 5 and the target plane is derived by using the imaging distance image acquired by the stereo camera 22. The information acquisition unit 51 need only acquire the imaging distance image. Hereinafter, derivation of the relative angle using the imaging distance image acquired by the stereo camera 22 will be described.

In a case where the relative angle is derived using the imaging distance image, the derivation unit 52 derives a difference in imaging distance between two or more points in the imaging distance image. In such a case, the derivation unit 52 detects a plane in the imaging distance image G2 and derives the difference only in the detected plane. FIG. 22 is a diagram illustrating the plane detection. As illustrated in FIG. 22, for a distance within a certain angle of view from the stereo camera 22 near the subject H on the patient table 30, the surface of the patient table 30 is flat and has a certain area, so that the imaging distances in the plurality of pixels are within a predetermined range, and a pixel group (indicated by black circles in FIG. 22) in which the imaging distances are within the predetermined range has an area equal to or larger than a certain value. On the other hand, the surface of the subject H is curved, and thus the imaging distances in the plurality of pixels (indicated by × marks in FIG. 22) representing the surface of the subject H exceed the predetermined range. The predetermined range can be, for example, ±2 cm.

Therefore, in a case where the imaging distances in the plurality of pixels are within the predetermined range and the pixel group in which the imaging distances are within the predetermined range has an area equal to or larger than a certain value in the imaging distance image G2, the derivation unit 52 determines that the pixel group constitutes the plane. The determined plane is the surface of the patient table 30. The derivation unit 52 derives the difference only on the determined plane. Since the pixel group in the predetermined range is determined to be the plane in a case where the pixel group has an area equal to or larger than a certain value, it is possible to prevent the detection of a small object having a flat surface as the plane in a case where the small object is near the patient table 30.

The derivation unit 52 can derive the relative angle of the detected plane, that is, the plane of the patient table 30 with respect to the radiation source unit 5 based on the magnitude of the difference between two or more positions derived in the plane of the imaging distance image G2. In this case, an accurate numerical value of the relative angle is not known, but the magnitude of the relative angle can be determined in accordance with the magnitude of the difference.

The display controller 53 may display the angle indicator on the display 25 in the same manner as in the above-described embodiment in accordance with the magnitude of the relative angle derived by the derivation unit 52 in this way.

In the above-described embodiment, the derivation unit 52 detects the plane in the imaging distance image G2 and derives the difference only in the detected plane, but the present disclosure is not limited to this. The difference may be derived for an entire region of the imaging distance image G2.

In the above-described embodiment, the radiation source unit 5 includes the optical camera 21 and the stereo camera 22 as separate bodies, but the present disclosure is not limited to this. A camera may be used in which the optical camera and the stereo camera are built in one housing.

In the present embodiment, each processing is executed by any computer. Also, any computer may execute these processes by a processor as hardware, a program as software, or a combination thereof. In this case, the processor is configured to execute various types of processing in the present embodiment in cooperation with the program and can function as each unit or each means in the present embodiment. Furthermore, the execution order of the processing by the processor is not limited to the above-described order, and may be changed as appropriate. Any computer may be a general-purpose computer, a computer for specific use, a workstation, or another system that can execute each processing.

The processor may be configured by one or more hardware components, and the type of hardware is not limited. For example, the processor may be configured by hardware, such as a central processing unit (CPU), a micro processing unit (MPU), a programmable logic device, such as a field programmable gate array (FPGA), a dedicated circuit that is used to execute specific processing, such as an application-specific integrated circuit (ASIC), a graphics processing unit (GPU), or a neural processing unit (NPU). Furthermore, the type of hardware may be a combination of different types of hardware components. In a case where the plurality of hardware components are configured to execute one or a plurality of types of processing of a certain processor, the plurality of hardware components may be present in devices physically separated from each other or may be present in the same device. Additionally, in any embodiment, the order of each processing by the processor is not limited to the order described above and may be changed as appropriate. In addition, the hardware is configured by an electrical circuit (circuitry) in which circuit elements, such as semiconductor elements, are combined.

Further, the program may be software such as firmware or microcode. Additionally, the program may be, for example, a program module group, and each function thereof may be executed by the processor configured to execute the corresponding function. The program may be a program code or a plurality of code segments stored in one or more non-transitory computer-readable media (for example, storage media or other storages). The program may be distributed and stored across a plurality of non-transitory computer-readable media existing in devices physically separated from each other. The program code or the code segment may represent a procedure, function, subprogram, routine, subroutine, module, software package, class, or any combination of instructions, data structures, or program statements. The program code or the code segment may be connected to another code segment or a hardware circuit by the transmission and reception of information, data, arguments, parameters, or contents in the memory.

In addition, in the above-described embodiment, the imaging support program 42 is stored (installed) in the storage 43 in advance, but the present disclosure is not limited to this. The imaging support program 42 may be provided in a form recorded on a recording medium, such as a compact disc read-only memory (CD-ROM), a digital versatile disc read-only memory (DVD-ROM), and a universal serial bus (USB) memory. In addition, the imaging support program 42 may be downloaded from an external apparatus through the network.

The disclosed technology is applicable to any program product. The program product includes all forms of products for providing the program. For example, the program product includes a program provided through a network such as the Internet, a non-transitory computer-readable recording medium such as a CD-ROM, a DVD, and a USB memory in which the program is stored and the like.

Hereinafter, supplementary notes of the present disclosure are set forth.

### Supplementary Note 1

An imaging support apparatus for a radiographic imaging apparatus including a radiation source that emits radiation, and an optical camera that is mounted on the radiation source and that captures an optical image in a direction from the radiation source toward a subject, the imaging support apparatus comprising: a display mounted on the radiation source; and a processor, in which the processor is configured to: derive a relative angle between the radiation source and a target plane; and display the optical image and an angle indicator representing the relative angle and a target angle on the display.

### Supplementary Note 2

The imaging support apparatus according to supplementary note 1, in which the display is mounted on the radiation source so as to rotate together with the optical camera.

### Supplementary Note 3

The imaging support apparatus according to supplementary note 1 or 2, further comprising: a first angle sensor that measures an angle of the radiation source; and a second angle sensor that measures an angle of the target plane, in which the processor is configured to derive the relative angle based on a difference between the angle of the radiation source measured by the first angle sensor and the angle of the target plane measured by the second angle sensor.

### Supplementary Note 4

The imaging support apparatus according to supplementary note 1 or 2, in which the processor is configured to: from a sensor that is provided on the radiation source and that acquires distance information representing an imaging distance in a direction from the radiation source toward the subject, acquire the distance information; and derive the relative angle based on a difference between the imaging distances at a plurality of positions in the direction from the radiation source toward the subject.

### Supplementary Note 5

The imaging support apparatus according to any one of supplementary notes 1 to 4, in which the target plane is a detection surface of a radiation detector that detects radiation transmitted through the subject to acquire a radiation image of the subject.

### Supplementary Note 6

The imaging support apparatus according to any one of supplementary notes 1 to 4, in which the target plane is a surface of a patient table on which the subject is placed.

### Supplementary Note 7

The imaging support apparatus according to any one of supplementary notes 1 to 6, in which the angle indicator includes a graphic for matching the relative angle to the target angle.

### Supplementary Note 8

The imaging support apparatus according to supplementary note 7, in which the angle indicator further includes an adjustment value for matching the relative angle to the target angle.

### Supplementary Note 9

The imaging support apparatus according to supplementary note 7, in which the graphic includes a first indicator representing the target angle and a second indicator representing the relative angle, and the processor is configured to change a position of the second indicator relative to a position of the first indicator in accordance with the relative angle.

### Supplementary Note 10

The imaging support apparatus according to supplementary note 9, in which the graphic includes a first linear graphic and a second linear graphic respectively for a vertical direction and a horizontal direction of the optical image, and the processor is configured to display the first linear graphic and the second linear graphic on edges of the optical image in the vertical direction and the horizontal direction.

### Supplementary Note 11

The imaging support apparatus according to supplementary note 7, in which the graphic has a predetermined reference shape, and the processor is configured to change the reference shape in accordance with the relative angle.

### Supplementary Note 12

The imaging support apparatus according to any one of supplementary notes 1 to 11, in which the processor is configured to further display an imaging center indicator representing an imaging center of the subject and an irradiation center indicator representing a center position of the radiation emitted from the radiation source on the display.

### Supplementary Note 13

An imaging support method for a radiographic imaging apparatus including a radiation source that emits radiation, and an optical camera that is mounted on the radiation source and that captures an optical image in a direction from the radiation source toward a subject, the imaging support method being executed by a computer, the imaging support method comprising: deriving a relative angle between the radiation source and a target plane; and displaying the optical image and an angle indicator representing the relative angle and a target angle on a display mounted on the radiation source.

### Supplementary Note 14

An imaging support program for a radiographic imaging apparatus including a radiation source that emits radiation, and an optical camera that is mounted on the radiation source and that captures an optical image in a direction from the radiation source toward a subject, the imaging support program causing a computer to execute: a procedure of deriving a relative angle between the radiation source and a target plane; and a procedure of displaying the optical image and an angle indicator representing the relative angle and a target angle on a display mounted on the radiation source.

### Supplementary Note 15

A radiographic imaging apparatus comprising: a radiation source that emits radiation; a sensor that acquires information representing an imaging distance in a direction from the radiation source toward a subject; an optical camera that captures an optical image in the direction from the radiation source toward the subject; and the imaging support apparatus according to any one of supplementary notes 1 to 12.

### Supplementary Note 16

The radiographic imaging apparatus according to supplementary note 15, further comprising: a body that is movable; and an arm that is foldable and that connects the body to the radiation source.

### Explanation of References

1: radiographic imaging apparatus
2: leg part
3: body
3A: housing
4: arm
5: radiation source unit
9: motion sensor
10: computer
11: leg
12: wheel part
13: handle
14: operation panel
15: first member
16: second member
17: mounting member
18: tube housing part
19: collimator
20: emission window
21: optical camera
22: stereo camera
22A, 22B: camera
23, 24: handle
25: display
30: patient table
31: radiation detector
32: motion sensor
41: CPU
42: imaging support program
43: storage
46: memory
45: I/F
47: network I/F
48: bus
51: information acquisition unit
52: derivation unit
53: display controller
60: display screen
61: information region
62: image region
63: indicator region
64, 69, 70: graphic
65: center region
66, 69A, 70A: mark
69B, 70B: line
71, 72: region
71A, 72A, 86: bar
73, 74, 75, 81A, 84, 85: indicator
76: frame
77, 77A, 77B: arrow
78, 79, 80: icon
81, 82: graphic
83: mark
G1: optical image
H: subject

## Claims

1. An imaging support apparatus for a radiographic imaging apparatus (1) including
a radiation source that emits radiation, and
an optical camera (21) that is mounted on the radiation source and that captures an optical image (G1) in a direction from the radiation source toward a subject (H), the imaging support apparatus comprising:
a display (25) mounted on the radiation source; and
a processor (41),
wherein the processor (41) is configured to:
derive a relative angle between the radiation source and a target plane; and
display (25) the optical image (G1) and an angle indicator representing the relative angle and a target angle on the display (25).

2. The imaging support apparatus according to claim 1,
wherein the display (25) is mounted on the radiation source so as to rotate together with the optical camera (21).

3. The imaging support apparatus according to claim 1 or 2, further comprising:
a first angle sensor that measures an angle of the radiation source; and
a second angle sensor that measures an angle of the target plane,
wherein the processor (41) is configured to derive the relative angle based on a difference between the angle of the radiation source measured by the first angle sensor and the angle of the target plane measured by the second angle sensor.

4. The imaging support apparatus according to claim 1 or 2,
wherein the processor (41) is configured to:
from a sensor that is provided on the radiation source and that acquires distance information representing an imaging distance in a direction from the radiation source toward the subject (H), acquire the distance information; and
derive the relative angle based on a difference between the imaging distances at a plurality of positions in the direction from the radiation source toward the subject (H).

5. The imaging support apparatus according to any one of claims 1 to 4,
wherein the target plane is a detection surface of a radiation detector (31) that detects radiation transmitted through the subject (H) to acquire a radiation image of the subject (H).

6. The imaging support apparatus according to any one of claims 1 to 4,
wherein the target plane is a surface of a patient table (30) on which the subject (H) is placed.

7. The imaging support apparatus according to any one of claims 1 to 6,
wherein the angle indicator includes a graphic for matching the relative angle to the target angle.

8. The imaging support apparatus according to claim 7,
wherein the angle indicator further includes an adjustment value for matching the relative angle to the target angle.

9. The imaging support apparatus according to claim 7,
wherein the graphic includes a first indicator representing the target angle and a second indicator representing the relative angle, and
the processor (41) is configured to change a position of the second indicator relative to a position of the first indicator in accordance with the relative angle.

10. The imaging support apparatus according to claim 9,
wherein the graphic includes a first linear graphic and a second linear graphic respectively for a vertical direction and a horizontal direction of the optical image (G1), and
the processor (41) is configured to display (25) the first linear graphic and the second linear graphic on edges of the optical image (G1) in the vertical direction and the horizontal direction.

11. The imaging support apparatus according to claim 7,
wherein the graphic has a predetermined reference shape, and
the processor (41) is configured to change the reference shape in accordance with the relative angle.

12. The imaging support apparatus according to any one of claims 1 to 11,
wherein the processor (41) is configured to further display (25) an imaging center indicator representing an imaging center of the subject (H) and an irradiation center indicator representing a center position of the radiation emitted from the radiation source on the display (25).

13. An imaging support method for a radiographic imaging apparatus (1) including
a radiation source that emits radiation, and
an optical camera (21) that is mounted on the radiation source and that captures an optical image (G1) in a direction from the radiation source toward a subject (H), the imaging support method being executed by a computer (10), the imaging support method comprising:
deriving a relative angle between the radiation source and a target plane; and
displaying the optical image (G1) and an angle indicator representing the relative angle and a target angle on a display (25) mounted on the radiation source.

14. A computer-readable storage medium that stores an imaging support program (42) for a radiographic imaging apparatus (1) including
a radiation source that emits radiation, and
an optical camera (21) that is mounted on the radiation source and that captures an optical image (G1) in a direction from the radiation source toward a subject, the imaging support program (42) causing a computer (10) to execute:
a procedure of deriving a relative angle between the radiation source and a target plane; and
a procedure of displaying the optical image (G1) and an angle indicator representing the relative angle and a target angle on a display (25) mounted on the radiation source.

15. A radiographic imaging apparatus (1) comprising:
a radiation source that emits radiation;
a sensor that acquires information representing an imaging distance in a direction from the radiation source toward a subject (H);
an optical camera (21) that captures an optical image (G1) in the direction from the radiation source toward the subject (H); and
the imaging support apparatus according to claim 1.

16. The radiographic imaging apparatus (1) according to claim 15, further comprising:
a body (3) that is movable; and
an arm (4) that is foldable and that connects the body (3) to the radiation source.
